# EUROPEAN PATENT SPECIFICATION

(11) **EP 1 380 206 B1**
(45) Date of publication and mention of the grant of the patent: **07.12.2005**
(21) Application number: 03023698.8
(22) Date of filing: 05.08.1997
(51) Int. Cl.: A01K 67/033

(54) **Aquaculture of marine worms**
Aquakultur von marinen Würmern
Aquaculture de vers marins

(30) Priority: 10.08.1996 GB 9616854
(43) Date of publication of application: 14.01.2004
(62) Divisional of application: 97934659.0
(73) Proprietor: Seabait Limited, Ashington, Northumberland NE63 9NW (GB)
(72) Inventor: Olive, Peter James William, Tyne and Wear NE26 4NS (GB)
(74) Representative: Ouzman, Beverley Nicola Claire

(56) References cited:
- US-A- 4 155 331
- DATABASE WPI Week 197742 Derwent Publications Ltd., London, GB; AN 1977-75170y XP002261699 & JP 52 107998 A (NIPPON NOKYO HYOSHI), 10 September 1977 (1977-09-10)

## Description

This invention relates to aquaculture of marine worms and particularly though not exclusively methods for the cryopreservation of the larvae of marine worms.

US-A-4,155,331 describes a method for the cryopreservation of multi-cellular organisms, especially immature crustaceans such as shrimp nauplii.

Marine bait worms are animals in the Class Polychaete of the Phylum Annelida or in the Phylum Sipunculida or are such other animals as may be generally referred to as worms which may be used as bait by anglers. Such worms are also used as feedstuffs for fish crustaceans and other organisms, for toxicity testing and for other scientific purposes.

Naturally occurring supplies of marine worms are not inexhaustible and collection of marine worms has been recognised as a cause of serious environmental concern.

Aquaculture of marine worms provides a sustainable source. However, the seasonal breeding cycle of marine worms hinders provision of a constant supply of worms throughout the year. Since the natural fecundity of female marine worms is very high, large numbers of fertilised eggs are available from time to time that are surplus to requirements and are usually wasted.

According to a first aspect, the present invention provides a method of preserving polychaete worms as recited in Claim 1.

According to a second aspect, the present invention provides a method of preserving polychaete worms as recited in Claim 3.

According to a third aspect, the present invention provides a method of recovering polychaete worms as recited in Claim 11.

The present invention also provides a method of aquaculture of polychaete worms according to claim 18.

Preferred features which may be used with aspects of the invention are set out in the dependent Claims.

The invention may be used as part of a worm breeding program. An example of such a program is set out below:

### Step 1 - Production of fertilised eggs

The larvae to be preserved by means of the invention may be derived from fertilisations of marine worms.

The fertilisation may be carried out by mixing fertilisable eggs (primary oocytes, secondary oocytes or later stages) with active spermatozoa at such concentrations as are suitable to bring about a fertilisation reaction in virtually all of the available eggs or by natural spawning.

The eggs to be fertilised may be suspended in sea water or in a salt solution of similar constitution or may be suspended in sea water after the fertilisation reaction has been carried out. The method of fertilisation and the stage of differentiation of the germ cells at the moment of fertilisation is known to vary between different species of worm. The method to be selected for the production of fertilised eggs must be appropriate for the species. The process may involve removal of germ cells from the body cavity of the male or the female worms when they have been observed to have cytological and physical characteristics known to be associated with the sexual and development maturity and ability to take part in a fertilisation reaction or secondly may involve treatment of the eggs with substances extracted from the nervous system or from other body tissues or with synthetic substances such as 8,11,14- eicosatrienoic acid which has been shown to have an effect on the processes of maturation of the germ cells of some species of worms.

### Step 2 - Rearing of the larvae to the stage of development suitable for preservation by means of the invention

The larvae to be preserved must be raised under conditions suitable for their development and will preferably have been grown in independent batches of larvae derived from one or a small number of fertilisations carried out at the same time according to the subsequent usage of the larvae which may require larvae of known parentage.

The larvae will preferably be at the same stage of development when selected for preservation by the process.

The larvae should be kept under clean though not necessarily sterile conditions in sea water or some similar and appropriate medium at densities sufficiently high to be economic but not sufficiently high as to cause mortality. Typically the larvae may be kept in shallow trays measuring 75 x 45 x 7 cm containing sea water to a depth of approximately 5 cm and containing approximately 50,000 larvae or alternatively may be suspended in plastic dust-bin like containers at densities of approximately 50,000 larvae per litre with gentle aeration or other method of stirring the sea water.

The larvae should preferably not be fed and should preferably be reared to the stage of development at which feeding is about to be initiated and the lipid and other food reserves in the gut tissues have been absorbed or utilised.

### Step 3 - Selection of the larvae and concentration for preservation

The larvae to be preserved should preferably be at a uniform stage of development as determined by microscopic examination. The larvae will preferably be at a stage of development where they have become capable of independent movement by cilia and/or by muscular action and may have reached a stage where three functional segments with erupted chaetae have formed but where the fourth segment has not developed to a stage where erupted chaetae are present and the larvae will preferably not have commenced feeding although the gut tissues may have reached a stage of development where the animals are about to begin feeding.

The larvae may be concentrated by pouring them in the sea water or other medium in which they have been developing onto a nylon mesh sieve or similar device with a mesh spacing of approximately 64 microns or such other dimensions as may be suitable to retain the larvae but allow free movement of the water. A suitable sieve can be made by gluing plastic mesh to the edges of a plastic beaker from which the base has been cut or some similar device may be used such that the larvae can be introduced into a series of solutions and removed from those solutions on the sieve.

The larvae will preferably be introduced on the sieve into a vessel containing a cryoprotectant mixture and from the sieve introduced into plastic straws, which may be coated with cholesterol or plastic bags, or foil bags or other such device as may be used with a controlled rate freezer.

The time at which the larvae are introduced to the device in which the larvae will be cooled will be noted and the total duration from the time of the first introduction to the time at which the cooling protocol is made to operate will be controlled and will be part of the operational procedure.

The cryoprotectant mixture may be one which contains various concentrations of Dimethyl-sulphoxide (DMSO) or 1,2 Propane diol with or without the further addition of non-penetrant organic agents which may aid the process and which may by preference be a solution of 1.5 Molar DMSO in sea water.

In the event that the cryoprotectant agent is a solution of 1.5 Molar DMSO in sea water the total time to elapse from the introduction of the larvae to the operation of the protocol shall not normally be greater than ten minutes and may by preference be not greater than four minutes.

### Step 4 - Preservation of the larvae by low temperature dehydration

The larvae will preferably be cooled at a predetermined rate that may normally be between 0.1 and 10 °C per minute with or without one or more periods of zero temperature change and the larvae will preferably be cooled to a temperature of between -25 and -35 °C depending on the rate of cooling used.

The larvae may be unfrozen and arranged in the presence of a frozen cryoprotectant mixture which thus leads to the dehydration of the larvae prior to the subsequent rapid cooling of the larvae as for example by plunging to larvae into liquid Nitrogen.

In the event that the cryoprotectant mixture contains 1.5 Molar DMSO in the presence of sea water, the temperature will preferably be such that less than 6% of the isotonic water content at equilibrium with sea water at 20 °C remains prior to the subsequent rapid cooling of the larvae as determined by the prior calculation of the volume of the larvae in equilibrium with an unfrozen solute with the osmolality characteristic of the terminal temperature with due allowance for the diffusion of water and permeability of the larvae.

Ragworm Nereis (Neanthes) virens larvae may be preserved when at their nechtochaete stage of development in which three well developed parapodia bearing chaetae operated by muscles are present.
The larvae will preferably be capable of movement and by preference they will not have initiated the formation of the fourth setiger or will have a rudimentary fourth setiger in which the chaetae have not erupted but may have developed beyond either of the above two stages of development. By preference they will also have developed the hind gut to the point where a lumen is present as indicated by a dark central line when observed through a microscope. Preserving the larvae at this stage in their development may enhance their ability to remain unfrozen in the presence of an ice cryoprotectant mixture, to be preserved and to be recovered.

### Step 5 - Transfer of the larvae to low temperature conditions and subsequent storage

The larvae may be transferred to a temperature of -196 °C by plunging them in devices such as straws, ampoules, bags and other such devices in which they have been cooled into a vessel containing liquid Nitrogen or by some other means that would achieve cooling rates necessary to prevent ice formation and that would allow for the permanent maintenance of the larvae at temperatures at or near -196 °C.

### Step 6 - Recovery of larvae from low temperature conditions

The larvae may be removed from the container at which they are maintained at or near -196 °C and held in a water bath at 20 °C or near that temperature for a short period until the frozen cryoprotectant mixture is observed to thaw. The larvae may be discharged from the device be it straw, ampoule, bag and other structure onto a sieve. The larvae may be introduced into a solution of sea water or other suitable medium of a volume approximately the same as the volume of cryoprotectant mixture in which the larvae were frozen and may be maintained in that solution for two minutes with gentle agitation prior to introduction of a second similar volume of sea water or similar medium for a further two minutes, after which time the DMSO or other cryoprotectant substance would be deemed to have diffused out of the larvae in sufficient quantity, and the larvae may be introduced into a large container of sea water suitable for the further growth of the larvae. The larvae may be washed twice by replacing the large volume of water into which they had been introduced with fresh sea water to remove final traces of DMSO or such cryoprotectant agents as might have been used. The larvae may then be maintained and reared to the stage at which they would be suitable for the specific purpose.

The invention may be used to control the time of year at which marine worms grow and develop.

Embodiments utilising the present invention and relating to preservation of marine worms are described, by way of example, in the following examples. The embodiments of the present invention that are described may be applied to the larvae of the polychaete worm Nereis (= Neanthes) virens commonly referred to in the UK as the King ragworm.

### Example 1 - Preservation of the larvae of Nereis (Neanthes) virens

### Step 1

Primary oocytes having a mean diameter greater than 190 microns and having a clear translucent halo and having first been tested with mature sperm to confirm that they had reached a stage of development where they were able to complete a fertilisation reaction and to subsequently develop to the nechotochaete stage were removed from the coelomic fluid of a female specimen of Nereis (Neanthes) virens raised from birth under conditions of commercial production at a temperature in the range 18-20 °C throughout life and under ambient photoperiod conditions.

The primary oocytes numbered approximately 300,000 were mixed with coelomic fluid taken from a male worm having dissociated spermatozoa which would become active and show forward movement following dilution in sea water in a volume of one litre.

### Step 2

Five minutes after the addition of the sperm containing fluid to the sea water containing the primary oocytes the sperm/oocyte mixture was further diluted with filtered sea water and transferred to a series of trays containing sea water to a depth of 3 cm and diluted such that the number of larvae did not exceed 5,000 larvae per m².

The larvae developed in the covered trays under a photoperiod of 12L:12D for a period of ten days at a temperature of 12 °C without feeding or the addition of food of any kind.

### Step 3

Samples of larvae were taken at random from the population of larvae developing in the trays and the stage of development determined by examination through a binocular microscope.

The larvae were observed to have a well developed gut with rather small lipid droplets in which the lumen can be distinguished as a distinct central line.

The larvae were observed to have three pairs of chaetigers with erupted chaetae and only a very rudimentary fourth chaetiger in which the chaetae had not erupted and the larvae had not started to feed.

The larvae were concentrated for the purposes of the process on a nylon mesh sieve which had been constructed by gluing 64 micron netting onto a rigid transparent plastic tube with a diameter of 2 cm and which was ideally suited for treating relatively small numbers of larvae.

### Step 4

The concentrated larvae were suspended in sea water at a concentration of about 1000 larvae per ml.

The larvae in sea water were mixed with a solution of DMSO in sea water such that the final concentration of the DMSO in sea water/DMSO mixture was 10% by volume. This was achieved by mixing one volume of sea water/larvae with three volumes of 13.34% DMSO by volume in sea water.

The mixture containing the larvae was drawn into 0.5 ml plastic straws and loaded onto a device for holding such straws and introduced into the cooling chamber of a controlled rate freezer. The total time elapsed from the first introduction of the larvae to the DMSO containing sea water mixture to the operation of the cooling process was three minutes.

The straws were taken to a start temperature of 20°C and then cooled according to the following protocol. The larvae were cooled from the start temperature of 20 °C at a rate of 5 °C per minute to a temperature of -5 °C and then cooled at a rate of 0.3 °C per minute to a temperature of -25 °C.

Three straws were introduced to the chamber of the controlled rate freezer during a period not greater than three minutes each straw and the larvae in them having been subjected to the same procedural steps. In this way each straw was considered to constitute a replicate treatment.

### Step 5

The straws were removed from the chamber of the controlled rate freezer and dropped into liquid Nitrogen in a glass lined vacuum vessel and after rapid cooling in this way introduced into a numbered container in a large dewar containing liquid Nitrogen in which the larvae were to be stored.

### Step 6

After one week, and on the same day, each of the three straws was removed from the numbered container and defrosted by holding the straw in a water bath at 20 °C until the ice core in the straw was observed to melt. The end of the straw was then cut and the liquid and larvae discharged into an equal volume of sea water in a glass vessel. After two minutes a volume of sea water equal to the volume of the combined cryopreservation mixture/sea water/larvae mixture was added. After a further two minutes the mixture was added to a large volume (about one litre) of sea water in a clear plastic aquarium tank. After two minutes the larvae were removed from this large volume of sea water by pouring them gently over a 64 micron sieve and added to a similar volume of fresh sea water. The procedure was repeated and batches of the larvae numbering approximately 200 larvae per batch were maintained in sterilised sea water at a density of about one larvae per ml in glass vessels at 12 °C for a period of seven days.

On the second day after feeding the larvae were fed by the introduction of 5 ml of a live culture of tetraselmis soeccia. The containers were stirred and observed daily and the water replaced and the larvae fed again on the third day.

The total number of larvae recovered from the straw was determined for each straw after three hours. After a period of seven days the total number of larvae capable of independent movement that were clearly alive and that were showing signs of further differentiation of the fourth setiger was determined and the survival rate calculated as the number of such larvae as a percentage of those observed to be present after three hours.

The results of these examinations are summarised in Table 1.

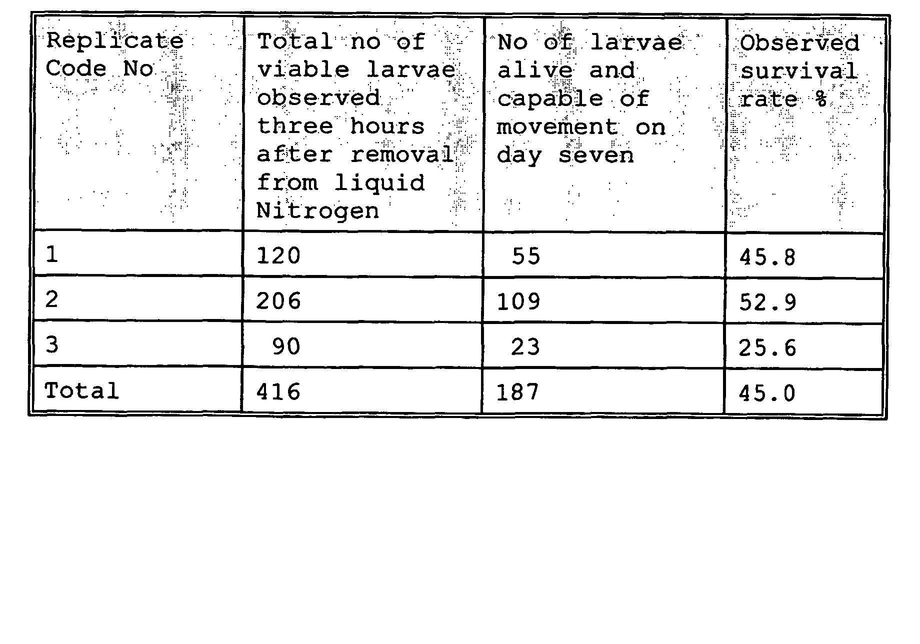

### Example 2 - Preservation of the larvae of Nereis (Neanthes) virens

### Steps 1 to 3

The larvae selected for Example 2 were identical in origin to those used in Example 1 and the same procedures were followed for Steps 1 to 3.

### Step 4

The concentrated larvae were suspended in sea water at a concentration of about 1000 larvae per ml.

The larvae in sea water were mixed with a solution of DMSO in sea water such that the final concentration of the DMSO in sea water/larvae/DMSO mixture was 10% by volume. This was achieved by mixing one volume of sea water/larvae with three volumes of 13.34% DMSO by volume in sea water.

The mixture containing the larvae was drawn into 0.5 ml plastic straws and loaded onto a device for holding such straws and introduced into the cooling chamber of a controlled rate freezer. The total time elapsed from the first introduction of the larvae to the DMSO containing sea water mixture to the operation of the cooling process was three minutes.

The straws were taken to a start temperature of 20°C and then cooled according to the following protocol. The larvae were cooled from the start temperature of 20 °C at a rate of 2.5 °C per minute to a temperature of -35 °C and then held at that temperature for one minute.

Three straws were introduced to the chamber of the controlled rate freezer during a period not greater than three minutes each straw and the larvae in them having been subjected to the same procedural steps. In this way each straw was considered to constitute a replicate treatment.

### Step 5

The straws were removed from the chamber of the controlled rate freezer and dropped into liquid Nitrogen in a glass lined vacuum vessel and after rapid cooling in this way introduced into a numbered container in a large dewar containing liquid Nitrogen in which the larvae were to be stored.

### Step 6

After one week, and on the same day, each of the three straws was removed from the numbered container and defrosted by holding the straw in a water bath at 20 °C until the ice core in the straw was observed to melt. The end of the straw was then cut and the liquid and larvae discharged into an equal volume of sea water in a glass vessel (solid clear glass watch-glass). After two minutes a volume of sea water equal to the volume of the combined cryopreservation mixture/sea water/larvae mixture was added. After a further two minutes the mixture was added to a large volume (about one litre) of sea water in a clear plastic aquarium tank. After two minutes the larvae were removed from this large volume of sea water by pouring them gently over a 64 micron sieve and added to a similar volume of fresh sea water.

This procedure was repeated and batches of the larvae numbered approximately 200 larvae per batch were maintained in sterilised sea water at a density of about one larvae per ml in glass vessels at 12 °C for a period of seven days.

On the second day after feeding the larvae were fed by the introduction of 5 ml of a live culture of tetraselmis soeccia. The containers were stirred and observed daily and the water replaced and the larvae fed again on the third day.

The total number of larvae recovered from the straw was determined for each replicate straw after three hours. After a period of seven days the total number of larvae capable of independent movement that were clearly alive and that were showing signs of further differentiation of the fourth setiger was determined and the survival rate calculated as the number of such larvae as a percentage of those observed to be present after three hours.

The results are summarised in Table 2.

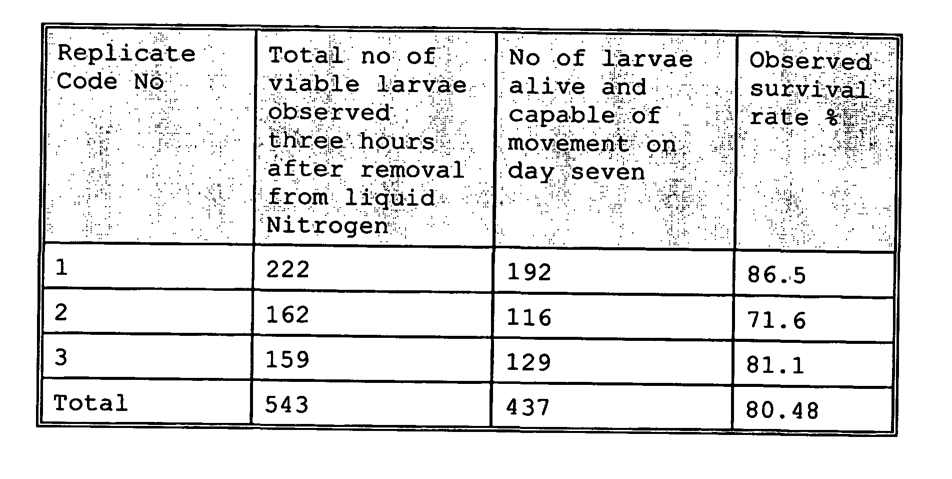

### Example 3 - Preservation of larvae of Nereis (Neanthes) virens and subsequent recovery from conditions suitable for commercial production

### Step 1

A female specimen of Nereis (Neanthes) virens raised from birth under conditions of commercial production at a temperature in the range 18-20 °C throughout life and under ambient photoperiod conditions and fed on a proprietary food was selected for the purposes of provision of larvae for preservation by means of the invention.

The primary oocytes of the female were observed by coelomic biopsy to have a mean diameter greater than 190 microns and having a clear translucent halo. A sample of the oocytes was removed by coelomic biopsy using a hypodermic syringe fitted with a suitable needle and mixed with a small sample of spermatozoa diluted in sea water to establish that the oocytes were capable of undergoing a fertilisation reaction as is characteristic for this species. The sample of oocytes was further maintained for a period of ten days to ensure that the fertilised eggs were capable of development to the stage were they had developed three pairs of parapodia with erupted chaetae (the nechtochaete stage) and this having been confirmed the females were used as a source of oocytes which after fertilisation would provide a source of juvenile worms for the purposes of the procedure.

The fertilisation was carried out in the following way:

A sample of approximately 2 ml of coelomic fluid was taken from a male in which dissociated spermatozoa were observed to be numerous in the body fluid. The sample of the coelomic fluid was introduced into a sample of sea water approximately 100 ml in volume. An aliquot of this sea water sperm mixture with a volume of about 25 ml was introduced into a sample of sea water such that the final volume was approximately 250 ml.

The primary oocytes of the selected female numbering approximately 750,000 in total were caused to be released into the sea water sperm mixture where they would come into contact with the spermatozoa and be fertilised virtually instantaneously.

A sample of the oocytes was examined to confirm that a high proportion of the oocytes (virtually 100%) exhibited the slightly elevated fertilisation membrane that is evidence that a fecund fertilisation reaction has taken place and the oocytes were diluted into a volume of five litres of sea water. Samples of this fertilised egg/sea water suspension were taken for the purposes of estimation of the total number of eggs present. The eggs were subsequently transferred to shallow trays in five litres of sea water at a density of 20,000 fertilised eggs per tray.

### Step 2

The larvae developed in the trays for a period of ten days at a temperature of 12 °C without feeding or the addition of food of any kind.

### Step 3

On the tenth day after having been fertilised the larvae were concentrated by pouring the sea water and larvae mixture through a sieve constructed by gluing 64 micron mesh to a suitable length of domestic UPVC piping with an internal diameter of 15 cm. A total of 70,000 larvae were so concentrated. A number of larvae suitable for introduction to a total of 12 x 0.5 ml plastic straws at a maximum density of 2,000 larvae per straw was introduced to a volume of 13.8% DMSO in sea water such that the final concentration of DMSO was 10% by volume and the resultant mixture was introduced into twelve plastic straws within a period of three minutes and loaded onto straw holders suitable for the appliance that was to be used for the purpose of cooling the larvae.

### Step 4

The straws were taken to a start temperature of 20°C and then cooled from the start temperature of 20°C at a rate of 2.5 °C per minute to a temperature of -35 °C and then held at that temperature for one minute.

### Step 5

The straws were removed from the chamber of the controlled rate freezer and dropped into liquid Nitrogen in a glass lined vacuum vessel and after rapid cooling in this way introduced into a numbered container in a large dewar containing liquid Nitrogen in which the larvae were to be stored.

### Step 6

After a period of one month (about thirty days) a total of thirty six straws containing cooled larvae were removed from liquid Nitrogen in batches of eight straws. Eight straws were defrosted at 20 °C in each batch treatment and the contents of the eight straws discharged into glass measuring vessel and an equal volume of sea water was added. After two minutes a further equal volume of sea water was added and after a further two minutes the mixture was poured over a nylon sieve and the larvae retained on the sieve washed into a large volume (about five litres) of sea water in the clear plastic aquarium tank. After two minutes the larvae were removed from this large volume of sea water by pouring them gently over a 64 micron sieve and added to a similar volume of fresh sea water. This procedure was repeated until a total of thirty six straws had been defrosted. After three hours the number of viable larvae was determined by counting the numbers of larvae found in a subsample of one micron taken from a litre of sea water in which the larvae had been dispersed by agitation.

The larvae were transferred to shallow trays at a density of 12,000 larvae per tray and approximately five litres of one micron filtered but non-sterile sea water per tray.

After two days the larvae were fed by the introduction of a quantity of Tetraselmis soeccia. The larvae were stirred and agitated by lifting the trays twice per day and the sea water was changed and the larvae re-fed on the third day.

The larvae were maintained at 12 °C for a period of seven days and after this period subsamples were taken to determine the approximate proportion of animals that had recovered and were apparently viable.

The subsamples showed that approximately 70,000 larvae had been recovered from liquid Nitrogen and that of these larvae approximately 35,000 had survived and were apparently viable having continued to be capable of movement, of feeding and commencing the process of chaetal sac differentiation in the fourth chaetiger.

On the seventh day after the initial recovery from liquid Nitrogen the surviving 35,000 larvae were transferred as a single batch to conditions suitable for their subsequent growth for commercial purposes for the supply as bait to the sea angling bait markets by introduction into a concrete bed with a total area of 2 m².

The larval worms were fed and maintained at 18-20 °C under long day (L:D 16:8) photoperiodic conditions.

After a period of seven weeks from the time that the larvae were introduced to the concrete tanks three sub-samples of the animals were taken by means of the insertion of a circular plastic core device with an area of 0.00785 m². The worms in the sub-samples were recovered by repeatedly shaking the sand mixture with sea water and pouring the mixture after a few minutes over a 64 micron mesh sieve as might be conveniently be used to recover small animals from a sand water mixture.

The number of animals recovered was determined. For two of the samples the mean length of the animals recovered when relaxed in 5% ethanol sea water was determined by measuring all of the intact animals present.

The number of segments present in the undamaged surviving animals was determined.

These observations were made for the purpose of establishing whether or not the animals had developed subsequently under conditions of culture suitable for commercial purposes such as for ongrowing for the purpose of supply of ragworms for use as bait by sea anglers and in a manner comparable to juvenile worms that had not been subjected to the preservation process.

The numbers of animals recovered are shown in Table 3.

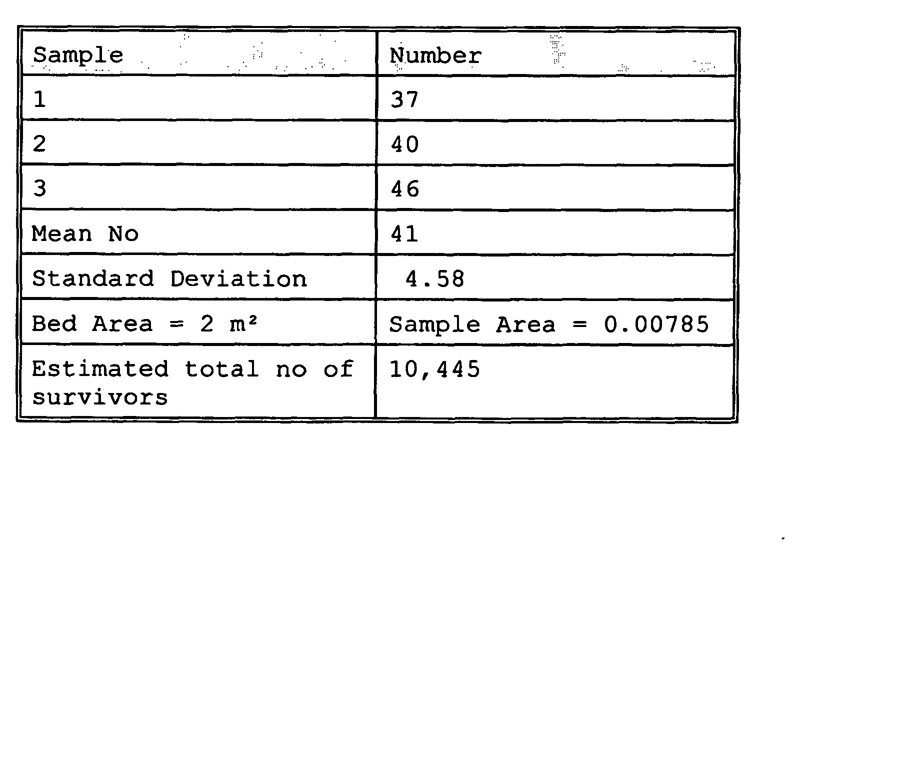

The data obtained by examination of the animals in samples 1 and 2 are shown in Table 4.

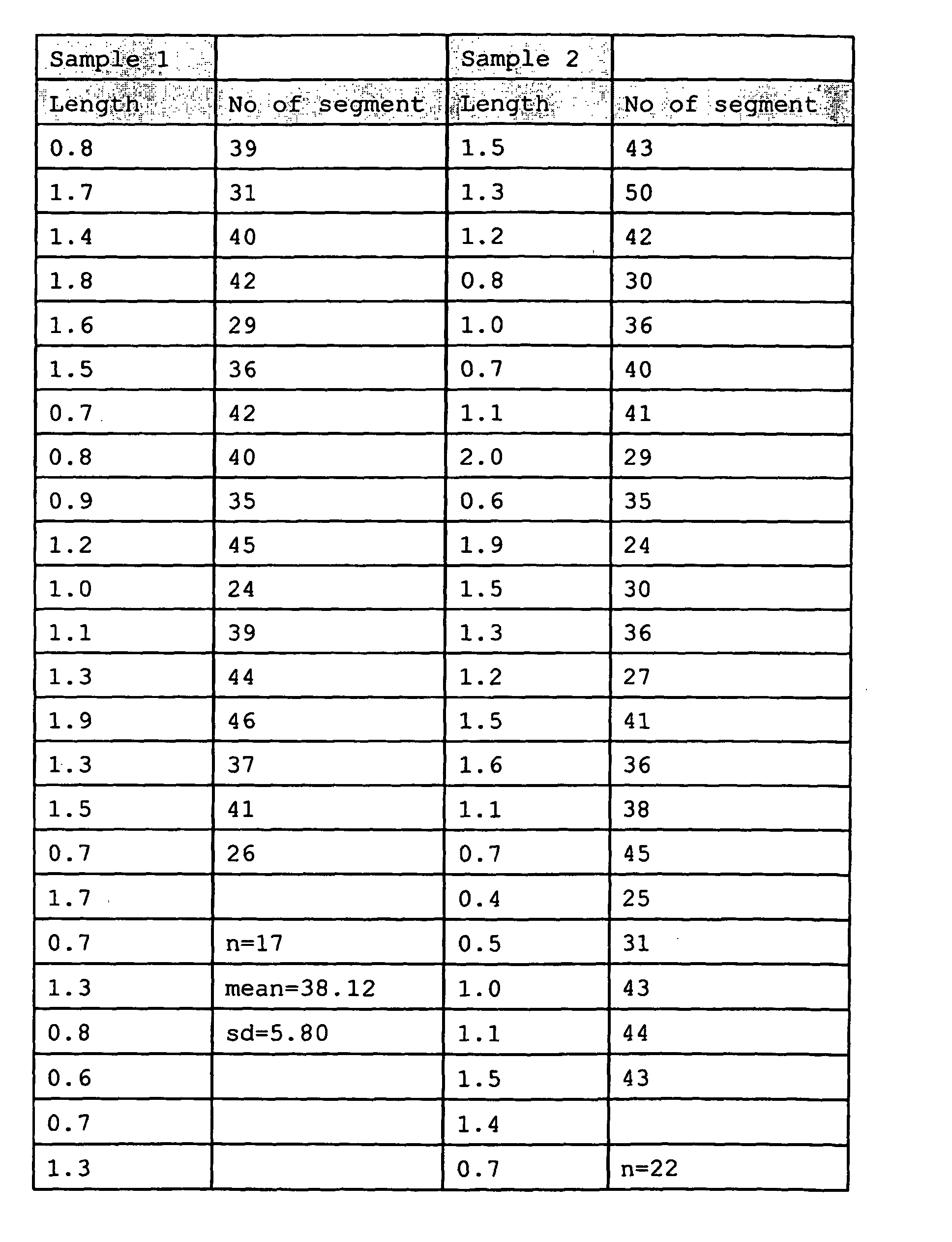

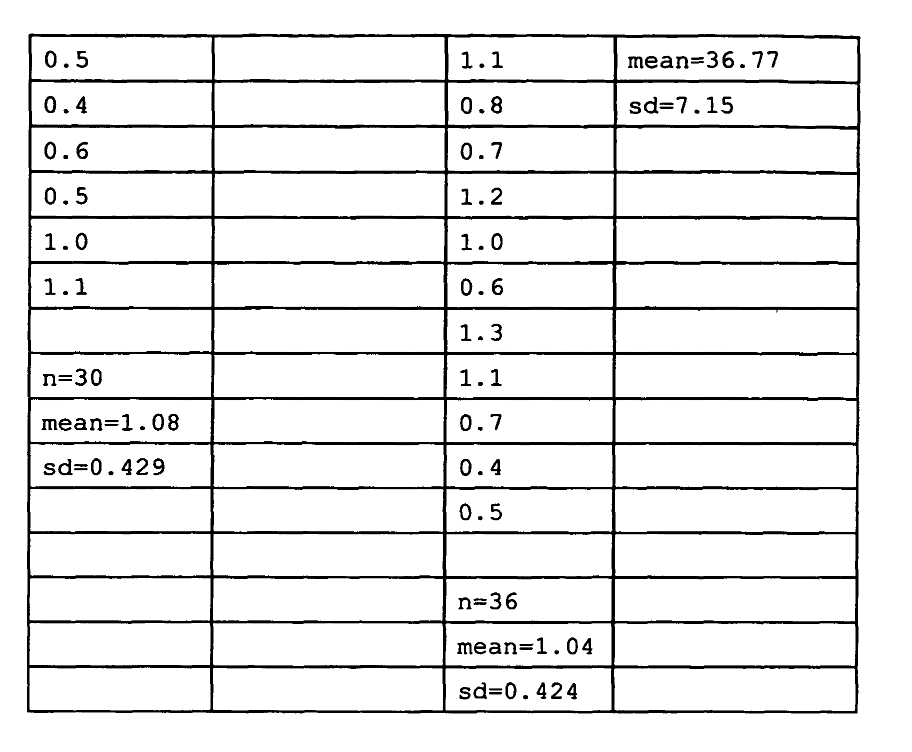

The rate of growth and the rate of segment proliferation indicate that the animals recovered from liquid Nitrogen had developed at a rate that is not different from that of animals otherwise produced growing under such conditions. The number of animals recovered indicates that the mortality of the animals has not been different from that of animals otherwise produced introduced at this age into similar conditions.

## Claims

1. A method of preserving polychaete (Polychaeta) larvae comprising rapidly cooling larvae that have developed to a stage at which they have three well developed parapodia bearing chaetae, and preserving or storing the larvae under cryogenic conditions.

2. A method in accordance with Claim 1 in which the larvae are preserved prior to reaching a stage of development at which their fourth segment has developed to an extent that erupted compound chaetae are present.

3. A method of preserving polychaete (Polychaeta) larvae comprising rapidly cooling larvae that have developed to a stage at which they have a differentiated gut with a lumen, and preserving or storing the larvae under cryogenic conditions.

4. A method in accordance with Claim 3 in which the larvae have not introduced food to the lumen of the gut.

5. A method in accordance with any preceding Claim in which the larvae are substantially without ingested food.

6. A method in accordance with any preceding Claim in which the larvae have used substantially all of the lipids in the gut.

7. A method in accordance with any preceding Claim in which the larvae are subjected to a low temperature dehydration prior to the rapid cooling.

8. A method in accordance with any preceding Claim in which the larvae are subjected to a process which leads to vitrification upon rapid cooling.

9. A method in accordance with Claim 8 in which the process is carried out at room temperature.

10. A method in accordance with any preceding Claim in which the rapid cooling is carried out by plunging the larvae into liquid Nitrogen.

11. A method of recovering larvae preserved by a method in accordance with any preceding Claim comprising removing the larvae from cryogenic storage.

12. A method in accordance with any preceding Claim in which the larvae are of worms which are members of the order Phyllodocida.

13. A method in accordance with Claim 12 in which the worms belong to the Nereidae family.

14. A method in accordance with Claim 13 in which the worms are of the species Nereis (Neanthes) virens.

15. A method in accordance with any one of Claims 1 to 12 in which the larvae are of worms which are members of the order of Eunicida.

16. A method in accordance with Claim 15 in which the worms belong to the family Eunicidae.

17. A method in accordance with Claim 16 in which the worms are members of the genus Marphysa.

18. A method of aquaculture of polychaete (Polychaeta) worms, wherein larvae of said worms are cryopreserved for a predetermined period of time according to any one of Claims 1 to 17, recovered from cryopreservation and allowed to develop into worms.

## Patentansprüche

1. Ein Verfahren zum Konservieren von Polychaete-Larven (Polychaeta-Larven), das das schnelle Abkühlen von Larven, die sich zu einer Stufe entwickelt haben, bei der sie drei gut entwickelte, Chaetae tragende Parapodien aufweisen, und das Konservieren oder Lagern der Larven unter Kryobedingungen beinhaltet.

2. Verfahren gemäß Anspruch 1, wobei die Larven konserviert werden, bevor sie eine Entwicklungsstufe erreichen, bei der sich ihr viertes Segment bis zu einem Ausmaß entwickelt hat, dass hervorgebrochene zusammengesetzte Chaetae vorhanden sind.

3. Ein Verfahren zum Konservieren von Polychaete-Larven (Polychaeta-Larven), das das schnelle Abkühlen von Larven, die sich zu einer Stufe entwickelt haben, bei der sie einen differenzierten Darm mit einem Lumen aufweisen, und das Konservieren oder Lagern der Larven unter Kryobedingungen beinhaltet.

4. Verfahren gemäß Anspruch 3, wobei die Larven keine Nahrung in das Lumen des Darms eingeführt haben.

5. Verfahren gemäß einem der vorhergehenden Ansprüche, wobei die Larven im Wesentlichen ohne eingenommene Nahrung sind.

6. Verfahren gemäß einem der vorhergehenden Ansprüche, wobei die Larven im Wesentlichen alle in dem Darm enthaltenen Lipide verbraucht haben.

7. Verfahren gemäß einem der vorhergehenden Ansprüche, wobei die Larven vor dem schnellen Abkühlen einer Niedrigtemperaturdehydration unterzogen werden.

8. Verfahren gemäß einem der vorhergehenden Ansprüche, wobei die Larven einem Vorgang unterzogen werden, der zu einer Vitrifizierung bei schnellem Abkühlen führt.

9. Verfahren gemäß Anspruch 8, wobei der Vorgang bei Zimmertemperatur durchgeführt ist.

10. Verfahren gemäß einem der vorhergehenden Ansprüche, wobei das schnelle Abkühlen durchgeführt wird, indem die Larven in Flüssigstickstoff eingetaucht werden.

11. Ein Verfahren zum Wiedergewinnen von Larven, die durch ein Verfahren gemäß einem der vorhergehenden Ansprüche konserviert worden sind, das das Entfernen der Larven aus der Kryolagerung beinhaltet.

12. Verfahren gemäß einem der vorhergehenden Ansprüche, wobei die Larven Larven von Würmern sind, die zu der Ordnung Phyllodocida gehören.

13. Verfahren gemäß Anspruch 12, wobei die Würmer der Familie Nereidae angehören.

14. Verfahren gemäß Anspruch 13, wobei die Würmer Würmer der Spezies Nereis (Neanthes) virens sind.

15. Verfahren gemäß einem der Ansprüche 1 bis 12, wobei die Larven Larven von Würmern sind, die zu der Ordnung Eunicida gehören.

16. Verfahren gemäß Anspruch 15, wobei die Würmer der Familie Eunicidae angehören.

17. Verfahren gemäß Anspruch 16, wobei die Würmer zu der Gattung Marphysa gehören.

18. Ein Verfahren zur Aquakultur von Polychaete-Würmern (Polychaeta-Würmern), wobei Larven dieser Würmer einen vorgegebenen Zeitraum lang gemäß einem der Ansprüche 1 bis 17 kryokonserviert werden, aus der Kryokonservierung wiedergewonnen werden und ihnen die Entwicklung zu Würmern ermöglicht wird.

## Revendications

1. Une méthode pour conserver des larves de polychètes (Polychaeta) comportant le refroidissement rapide de larves qui se sont développées jusqu'à un stade auquel elles ont trois parapodes bien développés portant des soies, et la conservation ou le stockage des larves dans des conditions cryogéniques.

2. Une méthode conformément à la revendication 1 dans laquelle les larves sont conservées avant d'atteindre un stade de développement auquel leur quatrième segment s'est développé jusqu'à un point où des soies en touffes ayant fait éruption sont présentes.

3. Une méthode pour conserver des larves de polychètes (Polychaeta) comportant le refroidissement rapide de larves qui se sont développées jusqu'à un stade auquel elles ont un intestin différentié avec une lumière, et la conservation ou le stockage des larves dans des conditions cryogéniques.

4. Une méthode conformément à la revendication 3 dans laquelle les larves n'ont pas introduit de nourriture dans la lumière de l'intestin.

5. Une méthode conformément à n'importe quelle revendication précédente dans laquelle les larves sont substantiellement sans nourriture ingérée.

6. Une méthode conformément à n'importe quelle revendication précédente dans laquelle les larves ont utilisé substantiellement tous les lipides présents dans l'intestin.

7. Une méthode conformément à n'importe quelle revendication précédente dans laquelle les larves sont soumises à une déshydratation à basse température avant le refroidissement rapide.

8. Une méthode conformément à n'importe quelle revendication précédente dans laquelle les larves sont soumises à un processus qui mène à la vitrification lors d'un refroidissement rapide.

9. Une méthode conformément à la revendication 8 dans laquelle le processus est mis en oeuvre à température ambiante.

10. Une méthode conformément à n'importe quelle revendication précédente dans laquelle le refroidissement rapide est effectué en plongeant les larves dans de l'azote liquide.

11. Une méthode pour récupérer des larves conservées par une méthode conformément à n'importe quelle revendication précédente comportant le retrait des larves du stockage cryogénique.

12. Une méthode conformément à n'importe quelle revendication précédente dans laquelle les larves sont des larves de vers qui sont membres de l'ordre des Phyllodocida.

13. Une méthode conformément à la revendication 12 dans laquelle les vers appartiennent à la famille des Nereidae.

14. Une méthode conformément à la revendication 13 dans laquelle les vers sont de l'espèce Nereis (Neanthes) virens.

15. Une méthode conformément à une quelconque des revendications 1 à 12 dans laquelle les larves sont des larves de vers qui sont membres de l'ordre des Eunicida.

16. Une méthode conformément à la revendication 15 dans laquelle les vers appartiennent à la famille des Eunicidae.

17. Une méthode conformément à la revendication 16 dans laquelle les vers sont membres du genre Marphysa.

18. Une méthode d'aquaculture de vers de polychète (Polychaeta), dans laquelle des larves desdits vers sont cryoconservées pendant un temps prédéterminé selon une quelconque des revendications 1 à 17, récupérées de la cryoconservation et autorisées à se développer pour devenir des vers.
